# EUROPEAN PATENT APPLICATION

(11) **EP 1 541 565 A1**
(43) Date of publication of application: **15.06.2005**
(21) Application number: 03028153.9
(22) Date of filing: 08.12.2003
(51) Int. Cl.: C07D 295/22, C07B 63/00, C07C 51/16, B01J 39/04

(54) **Process for the separation of organic nitrosonium and/or hydroxylamine compounds by means of cation exchange resins and recovery and oxidation processes based thereon**

(71) Applicant: SCA Hygiene Products AB, 405 03 Göteborg (SE)
(72) Inventor: Jetten, jan, 3701 JL Zeist (NL); Besemer, Arie, 3958 CC Amerongen (NL)
(74) Representative: HOFFMANN EITLE

(57) **Abstract**

The present invention relates to a process for the separation of a secondary organic nitrosonium compound and/or a secondary organic hydroxylamine compound from an acidic aqueous medium containing them in dissolved form, said process comprising the step of bringing into contact said acidic aqueous medium with a cation exchange resin, and the use of this process in a process for the recovery of secondary organic nitroxy compounds from an aqueous medium comprising said nitroxy compound and oxidation processes for hydroxy compounds.

## Description

The present invention relates to a process for the separation of organic nitrosonium and/or hydroxylamine compounds by means of a cation exchange resin, a recovery process for the corresponding nitroxy compounds based thereon as well as a process for the nitroxy-mediated oxidation of hydroxy compounds that also makes use of this separation process.

### Background art

Nitroxy-mediated oxidations are a useful tool to convert primary alcohols to aldehydes and/or carboxylic acids or secondary alcohols to ketones. Organic nitroxy compounds suitable for this purpose must be capable of forming stable radicals and therefore, as a rule, are secondary nitroxy compounds sterically shielded by at least one bulky group having a quaternary carbon atom in α-position to the nitrogen atom. Especially TEMPO (2,2,6,6-tetramethylpiperidine-N-oxyl formula I in the scheme below) and its derivatives have attracted a lot of interest over the last years since they allow the selective introduction of aldehyde and/or carboxylic acids into alcohol substrates such as polysaccharides.

The reaction can be conducted in three ways. First, a nitrosonium ion (III) which is the actual active species can be generated catalytically in situ from a nitroxide in the presence of a primary oxidant, such as hypochlorous acid/hypochlorite as shown in the scheme below. Depending on the process conditions, the nitrosonium ion will oxidize the alcohol, i.e. the hydroxy compound to the corresponding aldehyde (as shown in the scheme) or carboxy compound while being reduced itself to the hydroxylamine. Equimolar amounts of the resulting hydroxylamine and nitrosonium ion will then recombine (synproportionate) under suitable reaction conditions to form the nitroxy compound. Then, the catalytic cycle newly starts.

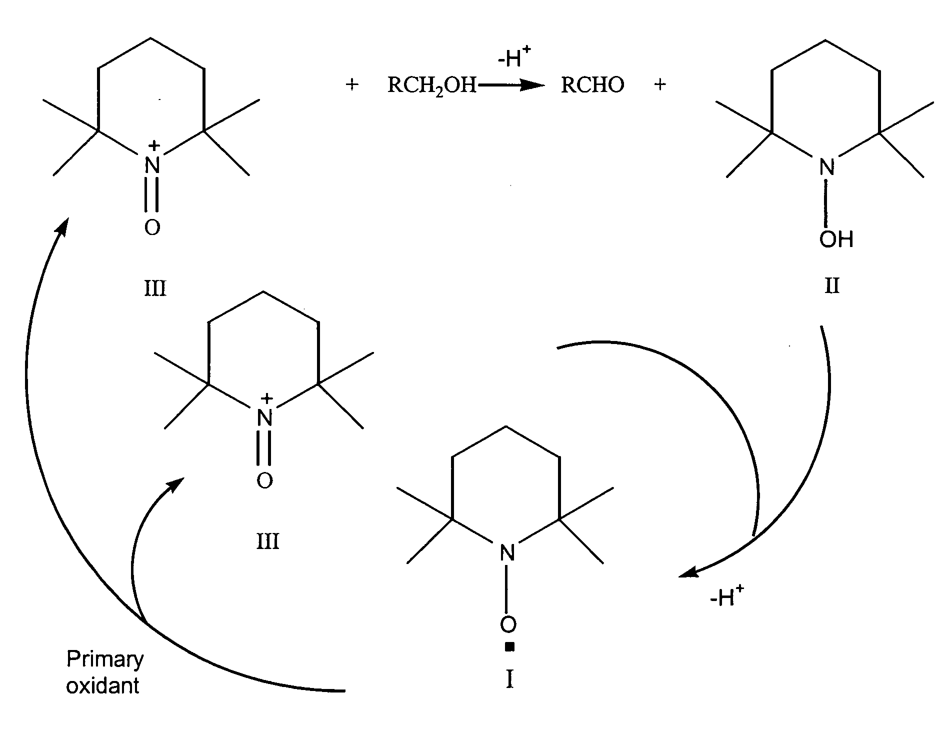

Second, the nitrosonium ion can be generated in situ by a disproportionation reaction of the nitroxy compound (I) to the corresponding hydroxylamine (II) and the nitrosonium ion (III) as shown in the following scheme. As the disproportionation reaction must be conducted under strongly acidic conditions, the hydroxylamine (II) is typically present in its protonated form (IV).

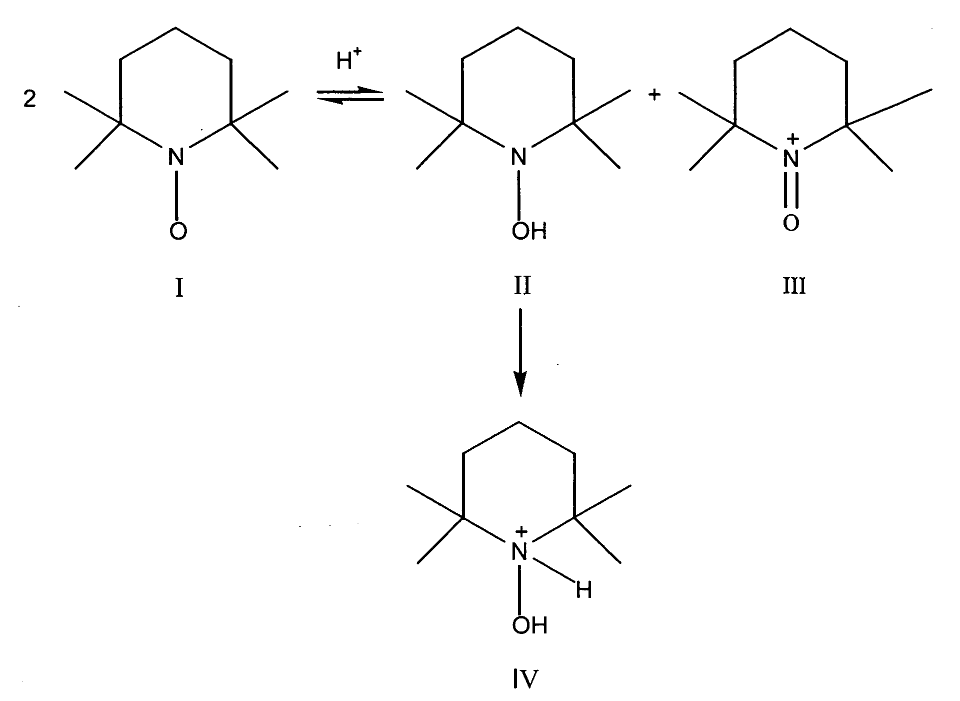

Third, it is also possible to prepare a salt of the nitrosonium ion, which then can be used in stoichiometric amounts.

Moreover, a relatively new technique involves the enzymatic conversion of nitroxy compounds as described in WO 00/50463.

There is extensive patent and scientific literature dealing with the synthesis and the use of nitroxy compounds, mostly TEMPO and its derivatives in oxidation reactions.

Reviews were published for instance by J.M. Bobbitt et al. in Heterocycles, vol. 27, No. 2, 1988 "Organic nitrosonium salts as oxidants in organic chemistry" and by A.E.J. De Nooy et al. in J. Synthetic Org. Chem 1996 (10),1153-1174 "on the use of stable organic nitroxyl radicals for the oxidation of primary and secondary alcohols".

If TEMPO and related nitroxy compounds are used as a catalyst in the oxidation of alcohols, for instance carbohydrates, the nitroxy compound is often lost during the isolation of the reaction product due to the relatively high volatility of nitroxy compounds such as TEMPO. This is an undesired situation for an economical process. Very few documents deal with technical solutions for overcoming this problem or the recovery of TEMPO in general.

WO 96/36621 proposes in this regard a method for obtaining a catalytically active mixture based on stable nitroxide radicals wherein at least a part of the reaction mixture is subjected to an azeotropic distillation. During the azeotropic distillation step volatile nitroxide radicals are at least partially distilled over with the liquid medium. However, this method requires distillation devices and creates high costs due to its energy consumption. Further, this method is not applicable to less volatile nitroxy compounds.

Another technique for avoiding the loss of TEMPO as a catalyst involves affixing the same to a polymer solid phase which then can be easily recovered from the reaction medium (T. Miyazawa, T. Endo et al. in J. Polymer Sci., Polymer Chem. Ed. 23 (1985) 1527-1535 and 2487-2494). Similarly, DE 42 09 869 A describes a method for immobilizing 4-hydroxy-TEMPO on polyvinyl-benzyl.

US 2003/0073871 pertains to the continuous oxidation of alcohol substrates to aldehydes in the presence of nitroxy compounds in an alkaline 2-phase system. The nitroxy compound can be present in the organic phase, in the aqueous phase or bound to solid supports.

However, the reaction rate of immobilized organic nitroxy compounds is typically lower than in homogeneous systems.

WO 02/59064 A1 relates to various recovery processes for TEMPO and related nitroxy compounds based on hydrophobic interactions. One option involves bringing the nitroxy compound into contact with hydrophobic resins such as XAD resins, for instance by passing the reaction mixture over a chromatographic column filled with one of these resins. The hydrophobic character of TEMPO and other organic nitroxy compounds leads to a strong affinity to hydrophobic resins allowing the separation from the reaction mixture. Nevertheless, this recovery process creates new problems insofar as, after eluting the organic nitroxy compound from the column, the organic solvent (eluent) has to be removed by evaporation. At this point in time, the high volatility of some organic nitroxy compounds such as TEMPO or 3,4-dehydro-TEMPO leads again to an undesired loss of this costly material. Furthermore, it would be desirable to recycle the organic nitroxy compound in aqueous media. Aqueous media are preferred for many nitroxy-mediated reactions and less hazardous than organic solvents.

WO 02/58844 describes a special group of nitroxy compounds wherein 4 TEMPO molecules are integrated into a heterocyclic molecule and their use in the oxidation of alcohol substrates. These nitroxy compounds can be recovered by filtration or water evaporation steps. One method for the recovery of the nitroxy compound catalyst involves the filtration at pH values above 8. According to another embodiment, the organic phase containing the catalyst is stirred with concentrated hydrochloric acid (HCl) to transfer the catalyst into the aqueous phase followed by evaporating the water under formation of the catalyst salt.

Further, it is known in the art that N-methylmorpholine-N-oxide (NMMO), i.e. a tertiary nitroxide, which is used as solvent in the Lyocell spinning process can be recovered and purified by means of anion exchange resins (H.Mertel et al., Papier (Darmstadt), 46 (3), 101-5, 1992, "Purification, recovery and methods for quantitative determination of N-methylmorpholine-N-oxide". The separation of tertiary amine oxides, such as NMMO from aqueous solutions by means of cationic exchange resins having carboxylate anchor groups is further known from EP 0 468 951 Al.

In view of the above, there is a strong need for an efficient recovery process for TEMPO, related organic nitroxy compounds and/or the catalytically active species thereof, i.e. the corresponding nitrosonium ion.

Therefore, it is one object of the present invention to provide a separation process for an organic hydroxylamine and/or organic nitrosonium ion compound which can be used in a recovery process for organic nitroxy compounds such as TEMPO and the corresponding nitroxy-mediated oxidation of hydroxy compounds.

It is one further object of the present invention to provide a separation process for organic nitrosonium and/or hydroxylamine compounds wherein organic solvents can be avoided.

It is a further object of the present invention to provide a process for the recovery of organic nitroxy compounds, such as TEMPO wherein the volatility of some nitroxy compounds does not cause any problems.

It is one further object of the present invention to provide a recovery process for organic nitroxy compounds with which the use of organic solvents can be avoided.

It is a further object of the present invention to provide a process for the oxidation of hydroxy compounds wherein the reaction mixture can be easily purified and the nitroxy mediator and/or the corresponding nitrosonium ion is easily recovered.

Finally, it is an object of the present invention to provide a process for the oxidation of a hydroxy compound that facilitates the recycling of various starting materials if these are not fully reacted.

Moreover, it is an object of the present invention to provide a closed cycle oxidation process for hydroxy compounds wherein the loss of organic nitroxy compound due to its volatility is minimised.

### Brief description of the invention

The above technical objects are solved by the following processes:
1. Process for the separation of a secondary organic nitrosonium compound and/or a secondary organic hydroxylamine compound from an acidic aqueous medium containing them in dissolved form, said process comprising the step of bringing into contact said acidic aqueous medium with a cation exchange resin.
2. Process for the recovery of secondary organic nitroxy compounds from an aqueous medium comprising said nitroxy compound, said process comprising the steps of:
   (a) acidifying the aqueous medium to disproportionate the nitroxy compound to the corresponding protonated hydroxylamine compound and nitrosonium compound,
   (b) contacting the mixture of protonated hydroxylamine compound and nitrosonium compound with a cation exchange resin to retain this mixture,
   (c) eluting the cation exchange resin with an aqueous solution of an acid or a metal salt in order to remove the hydroxylamine and nitrosonium compounds from the cation exchange resin,
   (d) neutralizing the resulting purified mixture of hydroxylamine and nitrosonium compounds to form the nitroxy compound.
3. Process for the oxidation of a hydroxy compound, said process comprising the steps of:
   (i) oxidizing a hydroxy compound in an aqueous medium in the presence of a secondary organic nitroxy compound and a primary oxidant,
   (ii) if necessary, acidifying the resulting reaction mixture to disproportionate unreacted nitroxy compound to the corresponding protonated hydroxylamine and nitrosonium ion followed by recovering the nitroxy compound in line with the above recovery process, which comprises contacting the mixture with a cation exchange resin,
   (iii) recycling the nitroxy compound recovered thereby to step (i).
4. Process for the oxidation of a hydroxy compound in the presence of an organic nitrosonium compound comprising the steps of:
   (i') oxidizing in an aqueous medium a hydroxy compound in the presence of a secondary organic nitrosonium compound while the nitrosonium ion compound is reduced to the corresponding protonated hydroxylamine,
   (ii') if necessary, acidifying the resulting oxidation mixture, followed by separating unreacted nitrosonium compound, if present, and hydroxylamine formed, in line with the above separation process which comprises contacting them with a cation exchange resin,
   (iii')eluting the unreacted nitroxy compound, if present, and the protonated hydroxylamine from the resin,
   (iv') adding a primary oxidant in order to convert the protonated hydroxylamine compound to the corresponding nitrosonium compound, and
   (v') recycling the nitrosonium compound to step (i').

### Detailed description of the invention

According to the claimed **separation process**, a secondary organic nitrosonium compound and/or a secondary organic hydroxylamine compound are brought into contact with a cation exchange resin retaining them. By virtue of the resulting interaction with the cation exchange resin, the nitrosonium and/or hydroxylamine compound which is protonated under acidic conditions can be separated from the acidic aqueous solution (e.g. reaction mixture) containing them and purified. As will be shown later, it is a major advantage of this separation process that the species to be separated are ionic (positively charged), in contrast to their precursor, i.e. the nitroxy compound, since ionic compounds forming a salt show a strong interaction with cation exchange resins and furthermore almost no volatility. This allows removing the aqueous medium while minimising the material loss.

It should be emphasized that the present invention is not restricted to particularly volatile nitroxy compounds such as TEMPO or 3,4-dehydro-TEMPO but can be equally used for less volatile species.

The separation process of the invention is conducted in an acidic aqueous medium (also referred to as "solution").

The pH value of this solution is preferably less than 4, more preferably less than 3, even more preferably less than 2 (throughout the specification and the claims pH values refer to a measurement at 20°C). Under these conditions, the existing equilibrium between organic nitroxy compound and hydroxylamine/nitrosonium ion will be shifted to the side of nitrosonium ion and hydroxylamine. Furthermore, the hydroxylamine is typical protonated under these acidic conditions, which entails a reduced volatility.

Accordingly, it is preferred that the acidic aqueous solution contains an organic nitrosonium and a protonated organic hydroxylamine compound, more preferably in equimolar amounts. However, the separation process of the present invention can also be successfully applied to acidic aqueous solutions containing only one of these species. Furthermore, according to one preferred embodiment, the mixture of nitrosonium and hydroxylamine compounds is obtained by disproportionating a secondary organic nitroxy compound under the above-explained acidic conditions.

To achieve this disproportionation reaction, any acid can be used (e.g. perchloric acid), although it is generally preferred to employ non-oxidising and non-reducing organic or inorganic acids for adjusting the pH to the above values. It is generally advantageous to use stronger acids, for instance those having pK values (at 25°C in an aqueous solution) of less than 5, preferably less than 3, for instance less than 1 or less than -1. Very strong acids fulfilling even the latter requirement are sulfuric acid or hydrochloric acid.

If recovery and oxidation processes, including the separation process of the invention, are to be conducted under TCF (total chlorine free) conditions, it is preferred to use chlorine-free acids, such as organic sulfonic acids (e.g. benzenesulfonic acid, o- or p- toluenesulfonic acid), sulfuric acid, phosphoric acid or nitric acid.

The type of acid used will also determine the counterion of nitrosonium and protonated hydroxylamine compound.

The phrase "aqueous solution" or "aqueous medium" as used in the specification and the claims refers preferably to water. However, it is also conceivable to use mixtures of water and a water-miscible organic solvent as a major and minor component, respectively. The amount of water-miscible organic solvent ranges preferably from 0 to 40 vol.%, e.g. 0 to 20 vol.%. Examples of suitable water-miscible organic solvents are alcoholic solvents such as ethanol, 1- or 2- propanol or t-butanol and in particular non-alcohol solvents such as ethers (e.g. dioxane or THF) or ketones (e.g. acetone) or nitriles (e.g. CH₃CN).

The acidic aqueous solution to be contacted with the cation exchange resin may also stem from a reaction mixture obtained by oxidising a hydroxy compound in the presence of a secondary organic nitroxy compound and/or the corresponding nitrosonium ion. The reaction can be subjected to filtration steps to remove insoluble matter before it is further purified by means of the cation exchange resin. The reaction mixture, as explained below in further detail, may contain unreacted hydroxy compound, oxidation products such as the resulting aldehyde and/or carboxy compound, unreacted nitrosonium ion, its reduced (consumed) form, i.e. the hydroxylamine, and/or nitroxy compounds. If this reaction mixture is brought into contact with a cation exchange resin, the organic nitrosonium and/or protonated hydroxylamine compounds will be subjected to a cation exchange reaction wherein the cation (typically hydrogen or an alkali metal ion, such as Na⁺) loosely bound to the cation exchange resin is displaced by the stronger binding organic nitrosonium and/or hydroxylamine compound. This exchange reaction allows removing the organic nitrosonium and/or hydroxylamine compounds from the reaction mixture.

As already indicated, the organic nitrosonium and/or hydroxylamine compound is preferably produced from a secondary organic nitroxy compound.

The term "secondary" means that the corresponding nitroxy compound is disubstituted by organic residues (This, as well as the following description of structural features, also naturally applies to the corresponding nitrosonium and hydroxylamine compounds). The organic nitroxy compound preferably has at least one quaternary carbon atom in α-position. The quaternary carbon atom causes sterical hindrance of the NO group thereby stabilizing the same. Preferably the organic nitroxy compound has two bulky groups (quaternary carbon atoms) in α-position. The NO functionality may be bound to optionally substituted phenol or aliphatic substituents that are linked to the nitrogen atom of the NO functionality by a quaternary carbon atom, e.g. tert-butyl. Moreover, it is preferred that both α-positions of the organic nitroxy compound are dimethyl-substituted.

It is similarly preferred that the nitroxy compound lacks □-hydrogen atoms. Two substituents can also be combined into an alkylen unit optionally interrupted by a hetero-atom (e.g. O,N) (to form an alicyclic or heterocyclic ring).

The total carbon number of the organic nitroxy compound is preferably less than 40, more preferably less than 30, in particular less than 20. The molecular weight is preferably less than 500.

It is preferred that the nitrogen atom of the NO group is linked to only two organic residues and not three as for instance in NMMO (N-methyl morpholin-N-oxide).

Preferred oxidation systems can be represented by the following formula (V) 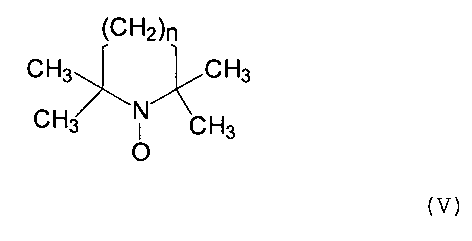 where n = 0 or 1 and where the methylene groups of the ring may carry one or more substituents selected from alkyl, alkoxy, aryl, aryloxy, amino, amido (e.g. acetamido, 2-bromacetamido and 2-iodacetamido), oxo, cyano, hydroxy, carboxyl, phosphonooxy, maleimido, isothiocyanato, alkyloxy, fluorophosphinyloxy (particularly ethoxyfluorophosphinyloxy), substituted or unsubstituted benzoyloxy, e.g. 4-nitrobenzoyloxy. If n = 1 (i.e. the ring represents a piperidine), these groups preferably substitute the 4-position of the piperidine. Examples are 4-acetamido-TEMPO, 4-acetoxy-TEMPO or 4-hydroxy-TEMPO. The di-tert.-alkyl nitroxy unit can also be present as part of a polymer structure such as {(CH₃)₂-C-(CH₂)₂₋₃-(CH₃)₂-C-NO-}ₘ-. Hydroxy, amino and amido are preferred among these substituents on account of the stability of the nitroxy compound under acidic conditions. The ring may also be partially unsaturated as in 3,4-dehydro-TEMPO.

An example of n=0 is PROXYL (2,2,5,5-tetramethylpyrrolidine-N-oxyl) .

Among the formula V compounds, the case of n=1 is preferred. This leads to the optionally substituted TEMPO compounds (2,2,6,6-tetramethyl-4-piperidine-N-oxide) that can selectively oxidize the primary hydroxy group at C(6) of the glucose unit of the cellulose into aldehyde and/or carboxyl groups.

DOXYL (4,4-dimethyloxazolidine-N-oxyl) can also be used as nitroxy compound in the present invention.

According to a preferred embodiment, the acidic aqueous solution containing the nitrosonium and/or hydroxylamine compound is contacted with the cation exchange resin by passing the aqueous solution over a chromatographic column filled with the resin. It is however also possible, to add the cation exchange resin to the acidic aqueous solution, for instance under stirring, followed by filtrating the resulting slurry. In this manner, the cation exchange resin, on which nitrosonium and/or hydroxylamine compounds are adsorbed, can be separated.

There are no specific limitations regarding the cation exchange resin to be used in the present invention. Commercially available cation exchange resins having anionic anchor groups such as sulfonate or carboxylate can be used. Suitable cation exchange resins have for instance an optionally crosslinked polystyrene/divinylbenzene-matrix or (meth)acrylic acid matrix to which the anionic anchor groups are linked. The cation exchange resin is preferably available in the form of small beads having an average particle size in the range of 30 µm to 1200 µm, preferably 35 to 300 µm.

Suitable, commercially available cation exchange resins involve strong acidic cation resin with 8% crosslinked gel cation, such as Dow 50 WX, Dow HCR-S; Rohm & Haas Amberlite IRP-120; Sybron C-249, C-267; Purolite C-100; Resintech CG8; IWT C-211; 10% crosslinked gel cation, such as Dow HGR; Rohm & Haas IR-122; Sybron C250, Purolite C-100x10; Resintech CG10; IWT C-361; or condensate polisher gel cation, such as Dow HCR-W2, HGR-W2; Rohm & Haas IR-130C, IR-132C; Sybron C-298, C-299; Purolite SGC-100x10 Dow HCR-W2, HGR-W2; Rohm & Hass IR-132C; Sybron C-298, C-299; purolite SGC-100x10; macroporous cation resin, such as Dow MSC-1, MAC-3, Rohm & Haas Amberlite 200, Amberlite 252; Sybron CFP-110; Purolite C-150; Resintech SACMP; IWT C-381; weak acid cation resin, such as Dow CCR-2, MWC-2; Rohm & Haas IRC-84, IRC-50, Sybron CCP; Purolite C-105, C-106; Resintech WACMP; IWT C-271, C-281; or uniform particle size cation resins, such as Dow Monosphere 650C or 750C, Marathon C and C-10; Rohm & Haas Amberjet 1200 and 1500; Sybron Impact CS-299 and CS-399 UPS Grades; Purolite Picopure 100 and 650C.

The products available from Dow Chemical are marketed under the trade name Dowex ®. Dowex ® 50WX has a styrene/divinylbenzene matrix and sulfonic acid groups. Dowex ® IDA-1 is characterized by a carboxylate-linked styrene/divinylbenzen matrix. Dowex ® monosphere 650C und 750C are styrene/divinylbenzene-based sulfonic acid resins. Dowex ® MAC-3 is macroporous polyacrylic acid based carboxy resin. Dow ® Marathon C represents again a styrene/divinylbenzene-based sulfonic acid resin.

Among these resins, strong acid cation resins, such as DOWEX 50WX (e.g. 50WX8) and Amberlite IRP-120 are preferred.

After being adsorbed on the cation exchange resin, the organic nitrosonium and/or hydroxylamine compound can be washed off or eluted, respectively, by means of suitable electrolyte-containing solutions. In this respect, it is recommendable to follow the instructions of resin manufacturer. Typically, an aqueous acid solution or the aqueous solution of a suitable metal salt can be used for this purpose. Suitable aqueous acids involve for instance 1 to 5 wt% HCl in water or 0.5 to 0.8 wt% H₂SO₄ in water. Preferably, metal salts showing a low affinity to the anion exchange resin, more preferably salts of a monovalent metal, in particular alkaline metal salts, such as sodium or potassium salts (e.g. their chlorides) are used. Typical and preferred concentrations of these salts range from 1 to 30 wt%. Higher concentrations of H⁺ or metal ions, as a rule, enhance the desorption from the column.

There are various manners of using the nitrosonium and/or hydroxylamine compounds desorbed from the cation exchange resin.

According to one preferred embodiment referring to mixtures of nitrosonium and hydroxylamine compounds, the eluted aqueous solution is neutralized, if necessary, to form the corresponding nitroxy compound by synproportionation. This synproportionation reaction starts to take place at pH values of at least 3, preferably at least 4, more preferably at least 5. The most preferred pH range for conducting this synproportionation reaction is 5 to 11. For this purpose any suitable, preferably non-oxidising and non-reducing base can be used, for instance monovalent metal bases, such as alkali metal bases or basic alkali metal salts such as NaOH, KOH, sodium or potassium carbonate, sodium or potassium hydrogen carbonate, sodium or potassium acetate. In this manner, the nitroxy compound, i.e. the starting material of organic nitrosonium and/or hydroxylamine is recovered and can be further used.

According to a second preferred embodiment, the hydroxylamine is directly oxidized to the corresponding nitrosonium ion, which represents the actual oxidizing species in nitroxy-mediated reactions. The resulting process variant allows generating the nitrosonium ion separately from the reaction mixture. This is advantageous insofar as a contact between the primary oxidant and the hydroxy compound substrate can be avoided if the latter is relatively sensitive to oxidation and tends to form undesired side products in the presence of a primary oxidant. The direct oxidation from the hydroxylamine to the corresponding nitrosonium ion can be achieved by strong oxidants, such as hypochlorous acid under acidic conditions where little or no synproportionation occurs (at a pH below 5, below 4 or below 3). Further suitable process conditions will be explained below, in the context of step (iv') of the second oxidation process of the invention.

The claimed **process for the recovery of secondary organic nitroxy compounds** comprises the following four steps:
(a) acidifying an aqueous solution containing the nitroxy compound to disproportionate the same to the corresponding protonated hydroxylamine compound and nitrosonium ion compound,
(b) contacting the mixture of protonated hydroxylamine compound and nitrosonium ion compound with a cation exchange resin to retain this mixture,
(c) eluting the cation exchange resin with an aqueous solution of an acid or a metal salt in order to remove the hydroxylamine and nitrosonium ion compounds from the cation exchange resin,
(d) neutralizing the resulting purified mixture of hydroxylamine and nitrosonium ion compounds until the nitroxy compound has formed.

As to the chemical structure of this nitroxy compound, reference can be made to the above description.

This applies as well to **step (a)**, as far as the conditions for acidifying the aqueous solution are concerned. The term "aqueous solution" is also understood in the same manner as above.

Step (b) of the recovery process according to the present invention involves contacting the mixture of protonated hydroxylamine and nitrosonium ion with a cation exchange resin under conditions that were already described in the context of the claimed separation process. The purpose of this step is to retain the mixture of hydroxylamine and nitrosonium ion on the cation exchange resin.

Step (c) of the recovery process involves eluting the cation exchange column with an aqueous solution of an acid or a metal salt in order to remove hydroxylamine and nitrosonium ion from the resin. A description of preferred embodiments of this eluent was already given in the preceding sections.

According to **step (d)** of the claimed recovery process, the resulting purified mixture of hydroxylamine and nitrosonium ion is neutralized until they recombine to the nitroxy compound by means of a synproportionation reaction. This synproportionation reaction starts to take place at pH values of at least 3, although it is preferred to adjust the pH to values of at least 4, preferably at least 5, in particular 5-11 in order to shift the equilibrium as far as possible to the nitroxy compound. Bases being suitable for neutralization were already mentioned.

According to one preferred embodiment of the claimed recovery process for nitroxy compounds, the aqueous solution subjected to steps (a)-(d) represents a reaction mixture obtained by oxidizing a hydroxy compound in the presence of a secondary organic nitroxy compound and/or the corresponding nitrosonium ion. As previously explained, this synthesis mixture may contain, apart from the nitroxy compound, primary oxidant and/or the reduced form thereof, nitrosonium and/or hydroxylamine compounds, unreacted hydroxy compound and its oxidation products as well as various side products which may for instance form by oxidative cleavage reactions. The recovery process of the present invention enables an efficient purification of this reaction mixture.

It is a specific merit of the recovery process according to the invention that the recovery steps for the nitroxy compound are performed with ionic compounds, i.e. the corresponding nitrosonium ion and protonated hydroxylamine salts. In contrast to the organic nitroxy compound, these are not volatile. Therefore, the nitroxy compound can be recovered without major material losses.

This recovery process can be used in a closed cycle oxidation process for a hydroxy compound in the presence of a secondary organic nitroxy compound and/or the corresponding nitrosonium ion. A **first embodiment** of the resulting **oxidation process** comprises the steps of:
(i) oxidizing a hydroxy compound in an aqueous medium in the presence of a secondary organic nitroxy compound **and a primary oxidant**,
(ii) if necessary, acidifying the resulting reaction mixture to disproportionate unreacted nitroxy compound to the corresponding protonated hydroxylamine and nitrosonium ion followed by recovering the nitroxy compound in line with previously described steps (a) to (d),
(iii) recycling the nitroxy compound recovered thereby to step (i).

As to **step (i)**, i.e., there is no specific limitation with respect to the type of hydroxy compound, primary oxidant and reaction conditions to be used.

It is one feature of nitroxy-mediated oxidations that they are compatible with a huge variety of different substrate hydroxy compounds. Thus, the hydroxy compound can comprise either a primary or secondary hydroxy functionality. The oxidation of a primary hydroxy compound is however preferred since techniques known in the art allow conducting the nitroxy-mediated oxidation of primary hydroxy compound with particularly high selectivity. The primary hydroxy compound is oxidized to the corresponding aldehyde and/or carboxylic acid. The interruption of the oxidation process and/or a suitable reaction conditions enable the isolation of the intermediate aldehyde. Interrupting the oxidation process is particularly suited for the oxidation of polysaccharides.

Examples for suitable hydroxy compound substrates are low molecular weight (up to MW 1000) aliphatic or aromatic-aliphatic hydroxy compounds, such as oligosaccharides as well as hydroxy compounds having a higher molecular weight including polymeric hydroxy compounds such as polysaccharides. The oxidation of oligo- and polysaccharides, such as starch or cellulose typically leads to the corresponding C6-aldehyde and/or carboxy derivates. The oxidation of cellulose and cellulose- containing materials, such as pulp is particularly preferred.

The starting pulps which may be used for oxidation may relate to primary fibrous materials (raw pulps) or to secondary fibrous materials, whereby a secondary fibrous material is defined as a fibrous raw material recovered from a recycling process. The primary fibrous materials may relate both to a chemically digested pulp (e.g. Kraft or sulfite pulp) and to mechanical pulp such as thermorefiner mechanical pulp (TMP), chemothermorefiner mechanical pulp (CTMP) or high temperature chemithermomechanical pulp (HTCTMP). Synthetic cellulose-containing fibers can also be used. Preference is nevertheless given to the use of pulp from plant material, particularly wood-forming plants. Fibers of softwood (usually originating from conifers), hardwood (usually originating from deciduous trees) or from cotton linters can be used for example. Fibers from esparto (alfa) grass, bagasse (cereal straw, rice straw, bamboo, hemp), kemp fibers, flax and other woody and cellulosic fiber sources can also be used as raw materials. The corresponding fiber source is chosen in accordance with the desired properties of the end product in a manner known in the art.

The oxidized pulps can be used for the manufacture of paper, in particular tissue paper having improved strength properties. Prior to or after oxidation the starting pulps can be beaten (refined) with the aim of further enhancing paper strength. In the manufacture of oxidized pulps it is further preferred to carry out all oxidation steps in the absence of chlorine-containing oxidants as basis for the production of TCF or ECF paper.

The hydroxy compound to be oxidized is preferably dissolved or dispersed in a suitable aqueous medium as defined above.

A list of suitable reaction conditions and compounds is found for instance in A.E.J. De Nooy, Synthesis 1996, 1153-1174). The pH range of the oxidation reaction generally varies between 1 and 14, preferably 2 and 12, particularly 4 and 11. The reaction temperature is preferably between 2°C and 80°C. The nitroxy compound may be added to the hydroxy compound as a solid (also as a pasty substance) or as a solution (usually as an aqueous solution). For mechanistical reasons, as known in the art, oxidation reactions performed at lower pH tend to proceed slower.

Preferably first the nitroxy compound and then the primary oxidizing agent is added. Suitable oxidants can be selected among primary oxidants typically used together with organic nitroxy compounds such as chlorine, bromine, iodine, hypochlorite, chlorite (in combination with hypochlorite), hypobromite, iodite, Fe(CN)₆³⁻ , transition metals of periods Va to VIIIa in the oxidation state of at least +3, oxidases, ozone, hydrogen peroxide, peroxosulfate and/or peracids. For each of the primary oxidants suitable reaction conditions, as known in the art, are to be selected. Thus, it can for instance be undesired to use hypchlorite at pH < 2 since then chlorine is formed, and tends to escape from the reaction mixture.

The oxidizing agent can be added all at once or distributed over the duration of the reaction (e.g. by evenly adding it in drops). The primary oxidizing agent (e.g. peracetic acid, ozone, hypohalite, the above metal compounds, enzyme) is preferably used in an amount of 1 to 100 mol%, in relation to the hydroxy functionality to be oxidized. The catalytic quantity of the nitroxy compound is preferably 0,01 to 5 mol% relative to the hydroxy functionality to be oxidized.

One version of performing the oxidation with the formula (V) nitroxy compound is described in WO 95/07393 which teaches the oxidation with a catalytic amount of the nitroxy compound and a hypohalite (e.g. NaOCl) as a primary oxidizing agent in an aqueous reaction medium at a pH between 9 and 13. Under these conditions, a primary hydroxy group (e.g. of cellulosic pulp) is oxidized via the corresponding aldehyde group into the carboxyl group.

An alternative version lies in the oxidation with a peracid, a precursor or a salt thereof as a primary oxidizing agent in the presence of a catalytic amount of the nitroxy compound (particularly optionally substituted TEMPO) and a catalytic amount of a halide (e.g. NaBr), preferably in the pH range of 2 to 11, particularly 2.5 to 3.5. The halide is preferably used in a quantity of 0.1 to 40, particularly 0.5 to 10 mol% relative to the hydroxy groups, e.g. those of cellulosic pulp. In the latter case, the nitroxy compound is preferably used in a quantity of 0.1 to 2.5 wt.%, relative to the dry weight (oven-dried) of the cellulosic pulp.
The peracid is preferably a peralkanoic acid, particularly peracetic acid, or persulfuric acid. Depending on the reaction duration, this embodiment of oxidation leads to aldehydes and/or carboxyl groups, for instance at C(6) of the glucose unit of the cellulose. It is, however, also possible to perform oxidation just using the nitroxy compound (particularly the formula (V) compound) as a mediator and peracid (e.g. persulfuric acid) as an oxidizing agent without halide, particularly bromide. More, preferred pH ranges are 6 to 8 for peracetic acid/bromide and 7 to 9 for persulfuric acid, respectively.

Another version lies in the combination of a catalytic amount of the nitroxy compound (particularly optionally substituted TEMPO) and a suitable oxidic compound of a metal of the transition metals of periods Va to VIIIa in the oxidation state of at least +3, e.g. oxides and oxygen-containing ions of manganese, chromium, iron, nickel, ruthenium and vanadium, e.g. vanadium pentoxide, iron oxide, chromium (VI) oxide, chromates and particularly manganese (IV) oxide and salts of permanganic acid. The reaction is preferably conducted at a pH between 2 and 8. The nitroxy compound is preferably used in a molar amount of 0,01 to 10%, preferably 0,05 to 5% based on the hydroxy functionality to be oxidized. As to pulp oxidation, an amount of 0.1 to 2.5 wt.%, relative to the dry weight (oven-dried) of the fibrous material is preferred. The reaction temperature is preferably less than 80°C, particularly 30 to 60°C. Depending on the duration of the reaction, this embodiment of the oxidation leads to aldehydes and/or carboxyl groups, for instance at C(6) of cellulosic pulp.

A further version lies in the oxidation of the cellulose with a catalytic amount of the formula (V) nitroxy compound that is hydroxy-, amino- or amido-substituted (e.g. 4-hydroxy TEMPO) at a pH between 1 and 7, particularly 2 to 6. In this version, a hypohalite (e.g. NaOCl) or ozone is particularly suitable as a primary oxidizing agent. The nitroxy compound is preferably used here in an amount of 4 to 20 mol% with respect to the hydroxy functionality to be oxidized. In the oxidation of pulp, the use of 0.05 to 15 wt.% nitroxy compound and 0.1 to 20 wt.% primary oxidizing agent, each relative to the dry weight (oven-dried) of the fibrous material is preferred. The reaction temperature is preferably 5 to 50°C. Depending on the reaction duration, this embodiment of oxidation results in aldehydes and/or carboxyl groups, e.g. at C(6) of the glucose unit of the cellulose. Halogen-free acids, such as sulfuric acid or toluenesulfonic acid, are particularly suitable for setting the pH.

According to a preferred embodiment, the nitroxy compound can also be used with enzymes and/or metal complexes. In this embodiment the catalytic amount of the nitroxyl compound is preferably 0,1-2,5 mol% with respect to the C(6)-alcohol to be oxidized.

The catalysts to be used according to this embodiment are oxidoreductases or other enzymes that are capable of oxidation in the presence of a suitable redox system. Oxido-reductases, i.e. enzymes capable of oxidation without the presence of further redox systems, to be used include peroxidases and oxidases, in particular polyphenol oxidases and laccase. Certain hydrolases, such as phytase, can be used when a further redox system is present such as a metal complex, e.g. vanadate. Metal complexes as such, without an enzyme protein, can also be used; examples include copper and iron complexes with porphyrins, phenanthrolins, polyamines such as EDTA, EGTA and the like. The metal-assisted enzymes and metal complexes require hydrogen peroxide, alkyl and ar(alk)yl hydroperoxides (such as tert-butyl hydroperoxide) or chlorite as an ultimate electron acceptor. Peroxidases (EC 1.11.1.1 - 1.11.1.11) that can be used according to the invention include the peroxidases which are cofactor-independent, in particular the classical peroxidases (EC 1.11.1.7). Peroxidases can be derived from any source, including plants, bacteria, filamentous and other fungi and yeasts. Examples are horseradish peroxidase, soy-hull peroxidase, myelo peroxidase, lactoperoxidase, Arthromyces and Coprinus peroxidases. Several peroxidases are commercially available. The peroxidases require hydrogen peroxide as an electron acceptor. Polyphenol oxidases (EC 1.10.3.1.) include tyrosinases and catechol oxidases such as lignine peroxidase. Suitable polyphenol oxidases may be obtained from fungi, plants or animals. The polyphenol oxidases require oxygen as an electron acceptor. Laccases (EC 1.10.3.2) are sometimes grouped under the polyphenol oxidases, but they can also be classified as a distinct group, sometimes referred to as p-diphenol oxidases. The laccases can be derived from plant sources or from microbial, especially fungal, sources. The laccases also require oxygen as an electron acceptor. The process for producing the aldehydes according to this embodiment can be performed under relatively mild conditions, e.g. at a pH between 2 and 10, and at a temperature between 15 and 60°C (both depending on the particular enzyme or metal complex). The reaction medium can be an aqueous medium, or a homogeneous mixed medium, e.g. a mixture of a water and a water-immiscible organic solvent such as a hydrophobic ether, a hydrocarbon or a halogenated hydrocarbon. In the latter case, the enzyme and/or the nitroxyl and the oxidising agent may be present in the aqueous phase and the hydroxy compound substrate and the aldehyde or ketone product may be present in the organic phase. If necessary, a phase transfer catalyst may be used. The reaction medium can also be solid/liquid mixture, in particular when the enzyme of the nitroxyl are immobilised on solid carrier. A heterogeneous reaction medium may be advantageous when the substrate or the product is relatively sensitive or when separation of the product from the other reagents may present difficulties.

According to the present invention it is preferred to conduct step (i), i.e. the oxidation of a hydroxy compound, with cellulosic pulp a hydroxy compound substrate in the presence of the above enzyme as primary oxidant and catalytic amounts of a nitroxy mediator, preferably those of formula (V), such as TEMPO or 4-acetamido TEMPO.

Regarding suitable reaction conditions for conducting the nitroxy-mediated oxidation of a hydroxy compound, reference can also be made to WO 95/07303, WO 99/57158, WO 99/58574, WO 00/26257, WO 00/50621, WO 00/50388, WO 00/50463, WO 01/00681, WO 00/50462, WO 01/34656, WO 01/34903, WO 01/34657, WO 01/83887, EP 1,149,846, EP 1,215,217 A and EP 1,251,140 A in the name of TNO and SCA Hygiene Products GmbH (AB, Zeist B.V).

**Steps (ii) and (iii)** are preferably conducted under the same conditions as outlined before. This extends as well to the term "aqueous" used to define the medium of step (i).

According to a **second embodiment** of the claimed oxidation **process**, the already formed **nitrosonium ion compound** is reacted with the hydroxy compound, rather than a mixture of nitroxy compound in catalytic amounts and primary oxidant. Thus, the generation of the nitrosonium ion via the oxidation by a primary oxidant takes place separately from the hydroxy compound substrate. This is of particular advantage if the hydroxy compound substrate tends to form undesired side products in the presence of a primary oxidant. This alternative embodiment comprises the following steps (i') to (v') :
(i') oxidizing in an aqueous medium a hydroxy compound in the presence of a secondary organic nitrosonium compound while the nitrosonium ion compound is reduced to the corresponding protonated hydroxylamine,
(ii') if necessary, acidifying the resulting oxidation mixture, and separating unreacted nitrosonium compound, if present, and hydroxylamine formed, in line with the above-described separation process which comprises contacting them with a cation exchange resin,
(iii')eluting the unreacted nitroxy compound, if present, and the protonated hydroxylamine from the resin,
(iv') adding a primary oxidant in order to convert the protonated hydroxylamine compound to the corresponding nitrosonium compound, and
(v') recycling the nitrosonium compound to step (i').

As to the hydroxy compound, the aqueous medium and the nitrosonium ion compound defined in **step (i')**, reference can be made to the above description. Moreover, there is no specific limitation regarding the pH to be used. It may however be of advantage, if the reaction is conducted under acidic conditions (e.g. pH below 4), since then no acidification is necessary in step (ii'), although the reaction then proceeds more slowly.

It should be added that the resulting hydroxylamine easily synproportionates with equimolar amounts of nitrosonium compound under weakly acidic, neutral or alkaline conditions thereby forming the nitroxy compound.

Further, in particular amido-substituted nitroxy compounds (e.g. 4-acetamido TEMPO) of the aforementioned formula (V) are suitable for step (i'). In this reaction, halogen-free acids such as sulfuric acid or toluenesulfonic acid are particularly suitable for adjusting the pH, preferably to values of 2 to 3. After the reaction, the consumed (reduced) form of the nitrosonium compound can be regenerated with ozone or another oxidizing agent, preferably in separate process step (iv'). An important advantage of the oxidation version discussed here is the ability to use the choice of a suitable pulp (TCF) to conduct the entire pulp or paper production method without any halogen-containing chemicals.

The nitrosonium compound is preferably used in an amount of 4 to 440 mol%, relative to the hydroxy functionality to be oxidized. This also includes the possibility of partially oxidizing polyhydroxy compounds such as cellulose present in pulp.

Regarding **steps (ii')** and **(iii')** the above-described conditions are fully applicable.

Primary oxidants suitable for **step (iv')** can be selected among primary oxidants typically used together with organic nitroxy compounds such as chlorine, bromine, iodine, hypochlorite, chlorite (in combination with hypochlorite), hypobromite, iodite, Fe(CN)₆³- , transition metals of periods Va to VIIIa in the oxidation state of at least +3, oxidases, ozone, hydrogen peroxide, peroxosulfate and/or peracids.

The oxidation is preferably conducted in an aqueous medium as afore-mentioned under conditions as explained for the first embodiment of the claimed oxidation process. It is however preferred to use acidic conditions involving a pH of preferably less than 4, more preferably less than 3, even more preferably less than 2 to suppress the synproportionation reaction between (not yet oxidized) hydroxylamine and already formed nitrosonium ion.

Step (v') of the second oxidation process of the invention closes the cycle by feeding the recovered nitrosonium compound back to step (i').

The present invention is now explained by means of the following example:

### Example 1

To 1 L of a solution containing 4g TEMPO, 15 ml of concentrated hydrochloric acid (37%) was added. The mixture was allowed to react for 30 minutes at room temperature. From this solution the absorbance at λ= 430 mm (characteristic for TEMPO)and at λ= 480 mm (characteristic for the corresponding nitrosonium salt) were measured and compared with the absorbance of the original solution. From the spectra it appeared that the mixture was disproportionated. 600 ml of the solution containing the protonated hydroxylamine (15 mmol) and nitrosnonium ion (15 mmol) was poured over a column containing 30 g cation exchanger (Dowex 50WX8). In the eluate the extinction of both TEMPO and its nitrosonium salt were measured (see Table 1). As can be seen, the eluted liquid contains only a limited amount of nitrosonium ion. To release the charged intermediates, the ion exchanger was washed with about 600 ml 1M hydrochloric acid. From this solution the absorbance at λ= 430 and at 480 nm were measured.

**Table 1:**

| Extinctions of TEMPO and its nitrosonium salt | | |
|---|---|---|
| Sample | Absorbance (430 nm)* | Absorbance (480 nm) |
| Acidified TEMPO solution prior to separation | 0.127 | 0.159 |
| Eluate from column | 0.024 | 0.005 |
| Eluate after washing with 1M HCl | 0.078 | 0.101 |

| | | |
|---|---|---|
| * This absorbance can be partly attributed to the presence of nitrosonium salt. | | |

From the data expressed in Table 1 it follows that the positively charged intermediates (protonated hydroxylamine and nitrosonium ion) initially bound to the cation exchanger material can be released from the column by elution with a high concentration of H⁺. After neutralization of eluted liquid, TEMPO was formed. Experiments in which the ion exchange material is eluted with Na⁺ gave the same results.

## Claims

1. Process for the separation of a secondary organic nitrosonium compound and/or a secondary organic hydroxylamine compound from an acidic aqueous medium containing them in dissolved form, said process comprising the step of bringing into contact said acidic aqueous medium with a cation exchange resin.

2. Process according to claim 1, wherein the acidic aqueous medium contains an organic nitrosonium and a protonated organic hydroxylamine compound.

3. Process according to claim 2, wherein the organic nitrosonium and hydroxylamine compounds are obtained by disproportionating a secondary organic nitroxy compound.

4. Process according to claim 1 wherein the acidic aqueous medium containing the organic nitrosonium and/or hydroxylamine compound is obtained from a reaction mixture obtained by oxidizing a hydroxy compound in the presence of a secondary organic nitroxy compound and/or the corresponding nitrosonium ion.

5. Process according to claim 3 or 4 wherein the organic nitroxy compound has the following formula (V): 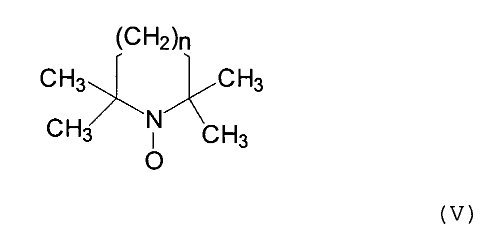 where n = 0 or 1 and where the methylene groups of the ring may carry one or more substituents selected from alkyl, alkoxy, aryl, aryloxy, amino, amido, oxo, cyano, hydroxy, carboxyl, phosphonooxy, maleimido, isothiocyanato, alkyloxy, fluorophosphinyloxy, substituted or unsubstituted benzoyloxy.

6. Process according to any of claims 1 to 5 wherein the step of contacting involves passing the aqueous medium over a column filled with a cation exchange resin.

7. Process according to any of claims 1 to 6 wherein the cation exchange resin has sulfonate and/or carboxylate anchor groups.

8. Process according to any of claims 1 to 7 wherein the organic nitrosonium and/or hydroxylamine compounds are recovered from the cation exchange resin by washing or eluting the resin with an aqueous acid solution or an aqueous solution of a metal salt.

9. Process according to claim 8 wherein the eluted aqueous medium contains organic nitrosonium and hydroxylamine, preferably in equimolar amounts and is neutralized to form the corresponding nitroxy compound.

10. Process according to claim 8 wherein the eluted aqueous medium contains at least the hydroxylamine which is oxidized to the corresponding nitrosonium compound.

11. Process for the recovery of secondary organic nitroxy compounds from an aqueous medium comprising said nitroxy compound, said process comprising the steps of:
(a) acidifying the aqueous medium to disproportionate the nitroxy compound to the corresponding protonated hydroxylamine compound and nitrosonium compound,
(b) contacting the mixture of protonated hydroxylamine compound and nitrosonium compound with a cation exchange resin to retain this mixture,
(c) eluting the cation exchange resin with an aqueous solution of an acid or a metal salt in order to remove the hydroxylamine and nitrosonium compounds from the cation exchange resin,
(d) neutralizing the resulting purified mixture of hydroxylamine and nitrosonium compounds to form the nitroxy compound.

12. Process according to claim 11 wherein the aqueous medium comprising said nitroxy compound was obtained from reaction mixture obtainable by oxidizing a hydroxy compound in the presence of a secondary organic nitroxy compound and/or the corresponding nitrosonium ion.

13. Process according to claims 11 or 12 wherein the organic nitroxy compound has the following formula (V): 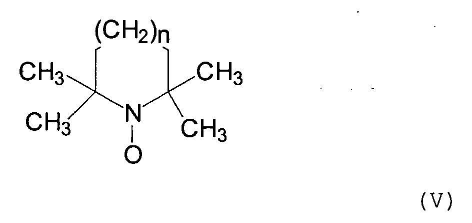 where n = 0 or 1 and where the methylene groups of the ring may carry one or more substituents selected from alkyl, alkoxy, aryl, aryloxy, amino, amido, oxo, cyano, hydroxy, carboxyl, phosphonooxy, maleimido, isothiocyanato, alkyloxy, fluorophosphinyloxy, substituted or unsubstituted benzoyloxy.

14. Process according to any of claims 11 to 13 wherein step (b) involves passing the aqueous medium over a column filled with a cation exchange resin.

15. Process according to any of claims 11 to 14 wherein the cation exchange resin has sulfonate and/or carboxylate anchor groups.

16. Process for the oxidation of a hydroxy compound, said process comprising the steps of:
(i) oxidizing a hydroxy compound in an aqueous medium in the presence of a secondary organic nitroxy compound and a primary oxidant,
(ii) if necessary, acidifying the resulting reaction mixture to disproportionate unreacted nitroxy compound to the corresponding protonated hydroxylamine and nitrosonium ion followed by recovering the nitroxy compound in line with steps (a) to (d) of claim 11,
(iii) recycling the nitroxy compound recovered thereby to step (i).

17. Process according to claim 16, wherein step (ii) is conducted in line with the processes according to any of claims 12 to 15.

18. Process for the oxidation of a hydroxy compound in the presence of an organic nitrosonium compound comprising the steps of:
(i') oxidizing in an aqueous medium a hydroxy compound in the presence of a secondary organic nitrosonium compound while the nitrosonium ion compound is reduced to the corresponding protonated hydroxylamine,
(ii') if necessary, acidifying the resulting oxidation mixture, followed by separating unreacted nitrosonium compound, if present, and hydroxylamine formed, in line with the process of claim 1 which comprises contacting them with a cation exchange resin,
(iii')eluting the unreacted nitroxy compound, if present, and the protonated hydroxylamine from the resin,
(iv') adding a primary oxidant in order to convert the protonated hydroxylamine compound to the corresponding nitrosonium compound, and
(v') recycling the nitrosonium compound to step (i').

19. Process according to claim 18, wherein step (ii) is conducted in line with the processes according to claims 2 to 10.

20. Process according to claim 16 or 18, wherein the hydroxy compound to be oxidized is cellulose present in cellulosic pulp.

21. Process according to claim 20 wherein the oxidation is conducted in the absence of chlorine-containing oxidants.
